# EUROPÄISCHE PATENTSCHRIFT

(11) **EP 0 470 469 B1**
(45) Veröffentlichungstag und Bekanntmachung des Hinweises auf die Patenterteilung: **13.03.1996**
(21) Anmeldenummer: 91112645.6
(22) Anmeldetag: 27.07.1991
(51) Int. Cl.: C07D 307/93

(54) **2-Oxa-3-alkoxy-7-hydroxy-bicyclo[2.2.1.]heptane**
2-Oxa-3-alkoxy-7-hydroxy-bicyclo[2.2.1]heptanes
Heptanes oxa-2-alkoxy-3-hydroxy 7-bicycliques[2.2.1]

(30) Priorität: 10.08.1990 DE 4025363
(43) Veröffentlichungstag der Anmeldung: 12.02.1992
(73) Patentinhaber: BASF Aktiengesellschaft, D-67063 Ludwigshafen (DE)
(72) Erfinder: Merger, Franz, Dr., W-6710 Frankenthal (DE); Witzel, Tom, Dr., W-6700 Ludwigshafen (DE); Becker, Rainer, Dr., W-6702 Bad Duerkheim (DE); Schindler, Gerhard, W-6800 Mannheim 1 (DE)

(56) Entgegenhaltungen:
- Tetrahedron Letters no. 25, Juni 1978, Great Britain Seiten 2165 - 2166; T.Akiyama et al.: "A Novel Stereoselective Synthesis of 1,2,3-trisubstitutedCyclopentanes"
- CHEMICAL ABSTRACTS, vol. 91, no. 2, 09 Juli 1979 Columbus, Ohio, USA& JP-A-54-19960[Kawanishi Kazumi et al.] 15 Feb.1979 Seite 415; Spalte 2; ref. no.11383G
- CHEMICAL ABSTRACTS, vol. 75, no. 21, 22 November 1971 Columbus, Ohio, USAWendelin,W.: "Heterocycles.33.1,4-Dialkyl-3-acet oxy-7-acetamido-2-oxabicyclo[2.2.1]heptanes." & Monatsh.Chem.1971,vol.102,no.4,p.1081-9 Seite 276; Spalte 1; ref. no. 129604C

## Beschreibung

Die vorliegende Erfindung betrifft neue 2-Oxa-3-alkoxy-7-hydroxy-bicyclo-[2.2.1]heptane der allgemeinen Formel I 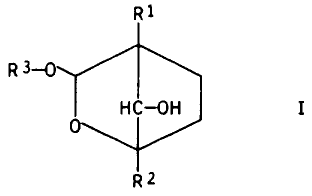 in der die Reste R¹, R und R³ folgende Bedeutung haben:
- C₁-C₁₀-Alkylgruppen, die bis zu drei der folgenden Substituenten R⁴ tragen können:
   C₃-C₁₂-Cycloalkyl; Phenyl; Naphthyl; 5- oder 6-gliedrige aromatische oder nichtaromatische Heterocyclen; C₂-C₆-Alkenyl; C₂-C₆-Alkinyl; C₁-C₁₂-Alkoxycarbonyl; C₁-C₁₂-Acyloxy; Amino; Mono-C₁-C₄-alkylamino; Di-C₁-C₄-alkylamino; C₁-C₁₂-Acyl; Di-(C₁-C₄-alkyl)-phosphonyl; Hydroxyl; Cyano; C₁-C₄-Alkoxy; C₁-C₄-Alkylthio; 5- oder 6-gliedrige Cycloalkylgruppen, deren Ringkohlenstoffatome durch nichtbenachbarte Sauerstoff- oder Schwefelatome ersetzt sein können;
- C₃-C₈-Cycloalkylgruppen,
- ein- oder zweikernige Arylreste, wobei diese bis zu drei C₁-C₁₂-Alkylgruppen, C₁-C₁₂-Alkoxygruppen, C₂-C₈-Alkenylgruppen, Chlor- oder Aminoreste als Substituenten tragen können,
- 5- oder 6-gliedrige aromatische oder nichtaromatische Heterocyclen, und in der die Reste R¹ oder R auch für Wasserstoff stehen können.

Außerdem betrifft die Erfindung ein Verfahren zur Herstellung der Verbindungen I, welches dadurch gekennzeichnet ist, daß man ein 2-Formyl-3,4-dihydropyran II mit einem Alkohol III in Gegenwart eines aciden Heterogenkatalysators bei 20 bis 350°C in der Flüssigphase umsetzt.

Weiterhin betrifft die Erfindung die Verwendung der Verbindungen I als Duftstoffe oder Zwischenprodukte zur Synthese von Wirkstoffen sowie die Verbindungen I enthaltende kosmetische Zubereitungen, Wasch-, Reinigungs- und Pflegemittel.

Aus Houben-Weyl, Bd. 6, Teil 4, S. 373-374 (1966) und J. Chem. Soc. 1398 (1953) sind Umsetzungen von 2,5-Dimethyl-2-formyl-3,4-dihydropyran mit Methanol bei 4 bis 20°C in Gegenwart von 2 mol-% Salzsäure zu 2-Dimethoxymethyl-2,5-dimethyl-6-methoxy-tetrahydropyran bekannt. Zusätzlich entstehen bicyclische Derivate wie 5,8-Dioxa-6-methoxybicyclo[3.2.1]-octan als Nebenprodukte. Die 8ildung von [2.2.1]-Bicyclen wurde jedoch nicht beobachtet.

Nach der älteren deutschen Anmeldung P 3 916 140.4 erhält man durch Umsetzung von 2-Formyl-3,4-dihydropyran an aciden Heterogenkatalysatoren bei Temperaturen von 50 bis 500°C 2-Formyl-cyclopentanone.

In der älteren deutschen Anmeldung P 3 916 138.2 ist die Reaktion von 2-Formyl-3,4-dihydropyranen mit Alkohol in Gegenwart acider Homogenkatalysatoren oder mit Wasser in Gegenwart acider Heterogenkatalysatoren in der Flüssigphase bei 150 bis 400°C zu Cyclopentanonen beschrieben.

Aus der P 3 916 139.0 ist die mit sehr hohen Ausbeuten verlaufende Umsetzung von 2-Formyl-3,4-dihydropyranen mit Alkoholen an aciden Heterogenkatalysatoren in der Gasphase bei 100 bis 500°C zu Cyclopentanonen bekannt.

Aus Tetrahedron Letters No. 25, Seiten 2165 - 2166 war ein Verfahren zur stereoselektiven Synthese von 1,2,3,-tri-substituierten Cyclopentanen durch Elektrolyse des Anions von 3-Methoxycarbonyl-7-oxabicyclo[2.2.1]heptan-2-carbonsäure, anschließende Wagner-Meerwein-Umlagerung des gebildeten 7-Oxa-bixcyclo[2.2.1]heptyl-2-cations unter Bildung des 2-Oxabicyclo[2.2.1]heptyl-3-kations, anschließende Methanolyse und Hydrierung bekannt.

Aufgabe der vorliegenden Erfindung war die Bereitstellung weiterer als Duftstoffe zu verwendender Verbindungen.

Demgemäß wurden die eingangs definierten 2-Oxa-3-alkoxy-7-hydroxy-bicyclo-[2.2.1]heptane gefunden.

Weiterhin wurde ein Verfahren zur Herstellung dieser Verbindungen, ihre Verwendung als Duftstoffe oder Zwischenprodukte zur Synthese von Wirkstoffen sowie die Verbindungen I enthaltene kosmetische Zubereitungen, Wasch-, Reinigungs- und Pflegemittel gefunden.

Die Reste R¹, R und R³ der erfindungsgemäßen 2-Oxa-3-alkoxy-7-hydroxy-bicyclo[2.2.1]heptane I bezeichnen C₁-C₁₀-Alkylgruppen, vorzugsweise C₁-C₈-Alkylgruppen, darunter vorzugsweise C₁-C₄-Alkylgruppen wie iso-Butyl, sec.-Butyl und tert.-Butyl und besonders n-Butyl, n-Propyl, iso-Propyl, Ethyl und vor allem Methyl.

Die Alkylgruppen können ihrerseits bis zu drei Substituenten R⁴ tragen, und zwar vorzugsweise:
- C₃-C₁₂-Cycloalkyl, vorzugsweise C₃-C₈-Cycloalkyl wie Cyclopropyl, Cyclobutyl, Cyclopentyl, Cycloheptyl, Cyclooctyl und besonders Cyclohexyl;
- Naphthyl und besonders Phenyl
- 5- oder 6-gliedrige aromatische oder nichtaromatische Heterocyclen wie vorzugsweise Pyridyl;
- C₂-C₆-Alkenyl, vorzugsweise C₂-C₄-Alkenyl wie Ethenyl, Propenyl und Butenyl;
- C₁-C₁₂-Alkoxycarbonyl, vorzugsweise C₁-C₈-Alkoxycarbonyl, darunter vorzugsweise C₁-C₂-Alkoxycarbonyl wie Ethoxycarbonyl und ganz besonders Methoxycarbonyl.

Daneben eignen sich als Substituenten R⁴
- vorzugsweise C₂-C₆-Alkinyl und C₁-C₁₂-Acyloxy;
- weiterhin Amino, Mono-C₁-C₄-alkylamino und Di-C₁-C₄-alkylamino;
- außerdem C₁-C₁₂-Acyl, Di-(C₁-C₄-alkyl)-phosphonyl, Hydroxyl, Cyano, C₁-C₄-Alkoxy, C₁-C₄-Alkylthio und 5- oder 6-gliedrige Cycloalkylgruppen, deren Ringkohlenstoffatome durch nichtbenachbarte Sauerstoff- oder Schwefelatome ersetzt sein können.

Weitere Bedeutungen der Reste R¹, R und R³ sind:
- vorzugsweise C₃-C₈-Cycloalkylgruppen wie besonders Cyclohexyl;
- weiterhin Arylreste wie vor allem Phenyl, wobei die Arylreste bis zu drei C₁-C₁₂-Alkylgruppen, C₁-C₁₂-Alkoxygruppen, C₂-C₈-Alkenylgruppen, Chlor- oder Aminoreste als Substituenten tragen können, vorzugsweise bis zu drei C₁-C₄-Alkylgruppen, C₁-C₄-Alkoxygruppen, C₂-C₄-Alkenylgruppen, Chlor- oder Aminoreste;
- außerdem 5- oder 6-gliedrige aromatische oder nichtaromatische Heterocyclen wie besonders Pyridyl.

Die Reste R¹ oder R können auch für Wasserstoff stehen, bevorzugt sind jedoch solche Verbindungen I, bei denen R¹ und R ungleich Wasserstoff sind, wobei hier wiederum solche Verbindungen besonders bevorzugt sind, bei denen R¹ und R die gleiche Bedeutung haben.

Die erfindungsgemäßen 2-Oxa-3-alkoxy-7-hydroxy-bicyclo[2.2.1]heptane I werden zweckmäßigerweise durch Umsetzung von 2-Formyl-3,4-dihydropyranen der allgemeinen Formel II 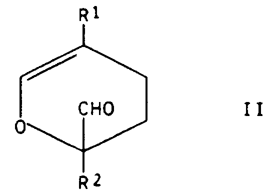 mit Alkoholen der allgemeinen Formel III

R³-OH III

in Gegenwart acider Heterogenkatalysatoren bei 20 bis 350°C in der Flüssigphase hergestellt.

Das erfindungsgemäße Verfahren läßt sich vermutlich wie folgt veranschaulichen:

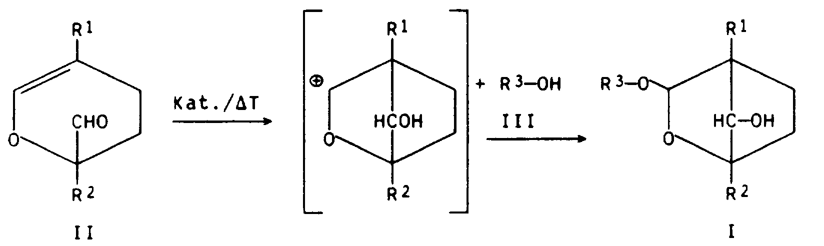

Die als Ausgangsverbindungen dienenden 2-Formyl-3,4-dihydropyrane II sind bekannt oder nach bekannten Methoden erhältlich, und zwar besonders einfach durch Erhitzen von α-substituierten Acroleinen nach Diels-Alder (Houben-Weyl, Bd. VII, Teil 1, S. 130-131; US-A-2,479,283; US-A-2,479,284):

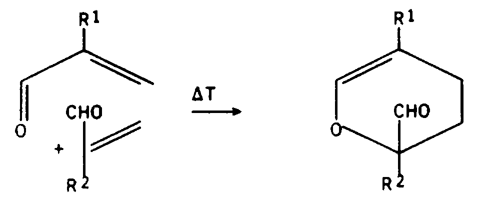

Die α-substituierten Acroleine können z.B. nach EP-A-58 927 durch Kondensation von Alkanalen mit Formaldehyd in hoher Ausbeute hergestellt werden.

Als Ausgangsstoffe II eignen sich:
- 2-Methyl-2-formyl-3,4-dihydropyran und
- 5-Methyl-2-formyl-3,4-dihydropyran.

Bevorzugte Ausgangsstoffe II sind solche mit zwei gleichen Resten R¹ und R in 2- und 5-Stellung:
- 2,5-Di-iso-propyl-2-formyl-3,4-dihydropyran
- 2,5-Di-n-butyl-2-formyl-3,4-dihydropyran
- 2,5-Di(methoxycarbonylmethyl)-2-formyl-3,4-dihydropyran
- 2,5-Di(1-methoxycarbonylethyl)-2-formyl-3,4-dihydropyran und
- 2,5-Di(3-methoxycarbonylpropyl)-2-formyl-3,4-dihydropyran.

Darunter besonders bevorzugte Verbindungen II sind:
- 2,5-Di-n-propyl-2-formyl-3,4-dihydropyran und
- 2,5-Di-ethyl-2-formyl-3,4-dihydropyran

Im Hinblick auf die gewünschten Verfahrensprodukte I ganz besonders bevorzugt ist 2,5-Dimethyl-2-formyl-3,4-dihydropyran.

Als erfindungsgemäß einzusetzende Alkohole III eignen sich C₃-C₆-Alkohole wie 2-Methyl-propanol-1, tert.-Butanol, 1-Methyl-butanol-1, 2-Methyl-butanol-1, n-Pentanol, Cyclopentanol, 2,5-Dimethylcyclopentanol, Cyclohexanol, aromatische Alkohole wie Phenol, Benzylalkohol und heteroaromatische Alkohole wie Furfurylalkohol.

Bevorzugt sind die C₂-C₅-Alkohole Ethanol, n-Propanol, iso-Propanol und 3-Methyl-butanol-1. Besonders bevorzugt ist Methanol.

Das Molverhälnis von II zu III beträgt im allgemeinen 0,1:1 bis 50:1, bevorzugt wird jedoch ein Molverhältnis von 0,5:1 bis 20:1, besonders von 1:1 bis 10:1.

Die Umsetzung von II mit III erfolgt durch Kontakt an aciden Heterogenkatalysatoren.

Als solche eignen sich Ionenaustauscher, Zeolithe und Phosphate wie Titan-, Aluminium- und Siliciumaluminiumphosphate.

Bevorzugt sind saure Oxide der II. bis V. Hauptgruppe des Periodensystems wie Boroxid, Aluminiumoxid, Zinndioxid und Siliciumoxid in Form von Kieselgel, Kieselgur und Quarz, Oxide der III. bis VII. Nebengruppe des Periodensystems wie Titandioxid und Zirkondioxid, Oxide der Seltenen Erdmetalle wie Ceroxid sowie Gemische dieser Oxide. Vorzugsweise können diese Katalysatoren noch durch Aufbringen von Zusätzen wie Phosphorsäure aktiviert werden. Besonders empfohlen werden Katalysatoren, die Siliciumdioxid enthalten und ganz besonders Siliciumdioxid.

Die Katalysatoren können wahlweise als 2- bis 4-mm-Stränge oder als Tabletten mit 3 bis 5 mm Durchmesser, als Splitt mit Teilchengrößen von 0,05 bis 1 mm, vorzugsweise 0,1 bis 0,5 mm als größten Durchmesser oder als Pulver mit Teilchengrößen von 0,1 bis 0,5 mm eingesetzt werden.

Die Menge der Katalysatoren, die man auch in ihren Mischungen einsetzen kann, wird bei diskontinuierlichen Verfahren vorzugsweise so bemessen, daß man 0,1 bis 20 Gew.-%, vorzugsweise 1 bis 10 Gew.-% des Katalysators zur Verfügung stellt, bezogen auf die Menge der Verbindung II.

Beim kontinuierlichen Verfahren beträgt die Katalysatorbelastung 0,01 bis 100 g, vorzugsweise 0,5 bis 50 g der Verbindung II pro Stunde und g des Katalysators.

Man führt die Umsetzung bei 20 bis 350°C, vorzugsweise bei 80 bis 300°C, und ganz besonders bevorzugt bei 130 bis 230°C durch.

Zweckmäßigerweise wird die Umsetzung bei Normaldruck vorgenommen, jedoch kann man auch bei vermindertem oder erhöhtem Druck arbeiten. Bevorzugt ist der Druckbereich von 1 bis 200 bar, besonders bevorzugt von 5 bis 150 bar.

Die Reaktionszeiten betragen normalerweise 10 Sekunden bis 3 Stunden, meistens 60 Sekunden bis 2 Stunden.

Es kann zweckmäßig sein, die Umsetzung unter Mitverwendung von inerten Lösungsmitteln durchzuführen. Hierbei eignen sich Kohlenwasserstoffe wie Dekahydronaphthalin oder Ether wie Diethylenglykoldiethylether.

Man kann die Reaktion diskontinuierlich in einem Rührreaktor, im allgemeinen unter Druck im Autoklaven, oder vorzugsweise kontinuierlich in einem Durchflußreaktor und in einer Rührreaktorkaskade vornehmen. So kommt eine Festbettreaktion im Sumpf- oder Rieselfahrweise in Betracht. Außerdem eignet sich eine Flüssigphasenreaktion mit suspendierten Trägerkatalysatoren.

Eine wirtschaftlich sehr vorteilhafte Ausführungsform des erfindungsgemäßen Verfahrens besteht in der kontinuierlichen Durchführung an einem Katalysatorfestbett, beispielsweise indem man eine Lösung aus 2-Formyl-3,4-dihydropyran II, dem Alkohol III und gegebenenfalls Lösungsmittel unter erhöhtem Druck über den festen Katalysator durch den auf die Reaktionstemperatur erhitzten Reaktor leitet, anschließend abkühlt und entspannt.

Der Verlauf der Reaktion kann mittels üblicher analytischer Methoden wie der Gaschromatographie verfolgt werden.

Als Nebenprodukte können neben den Ausgangsverbindungen 2-Formylcyclopentanone und Cyclopentanone gebildet werden, deren Anteil zweckmäßigerweise durch Wahl milderer Reaktionsbedingungen wie Temperaturabsenkung oder Erhöhung der Alkoholmenge oder Variation der Verweilzeit im Reaktor fast vollständig zurückgedrängt werden kann.

Die Aufarbeitung des Reaktionsgemisches auf die Verfahrensprodukte I nimmt man wie üblich vor, und zwar durch Phasentrennung, Extraktion und vorzugsweise durch fraktionierende Destillation.

Die nach dem erfindungsgemäßen Verfahren auf einfache und wirtschaftliche Weise erhältlichen 2-Oxa-3-alkoxy-7-hydroxy-bicyclo[2.2.1]heptane I eignen sich selber als Duftstoffe oder können als Zwischenprodukte zur Synthese von Wirkstoffen verwendet werden.

### Beispiele 1-9

Herstellung von 2-Oxa-3-alkoxy-7-hydroxy-bicyclo[2.2.1]heptanen der Formel 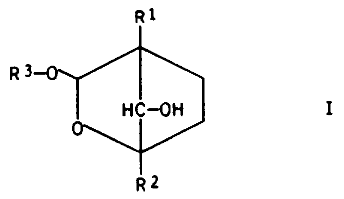 a g einer Lösung aus b Gew.-% eines 2-Formyl-3,4-dihydropyrans II und c Gew.-% eines Alkohols III wurden pro Stunde bei 90 bar und d°C durch ein mit 3,0 g Siliciumdioxid-Strängen gefülltes Rohr von 6 mm Durchmesser geleitet. Nach Abkühlung und Entspannung auf Normaldruck fielen stündlich e g Reaktionsgemisch an, welches gemäß quantitativer gaschromatographischer Analyse (innerer Standard) f Gew.-% Produkt I enthielt. Durch anschließende fraktionierende Destillation erhielt man das reine Produkt I als Diasteromerengemisch.

Die Einzelheiten dieser Versuche sowie deren Ergebnisse sind der nachstehenden Tabelle zu entnehmen.

### Beispiel 9

### Parfümöl

Es wurde ein Parfümöl aus folgenden Komponenten hergestellt:
100 g 2-Oxa-3-ethoxy-1,4-dimethyl-7-hydroxy-bicyclo[2.2.1]heptan
150 g Orangenöl süß Florida
120 g Hexylbenzoat
110 g Linalylacetat
70 g Linalool
70 g 3-(4-Methylphenyl)2-methylpropanol
100 g Diethylmalonat
60 g Phenylethylalkohol
60 g Citronellol
35 g Dimethylbenzylcarbinylacetat
10 g gamma-Undecalacton
20 g Dimethylheptanol
55 g Benzylacetat
30 g Phenylethylacetat
10 g Cyclohexylethylacetat.

Dieses Parfümöl ließ sich zur Herstellung von Parfüms, Eau de Toilettes, Seifen und sonstigen kosmetischen Zubereitungen mit einer angenehmen fruchtig-blumigen Duftnote verarbeiten.

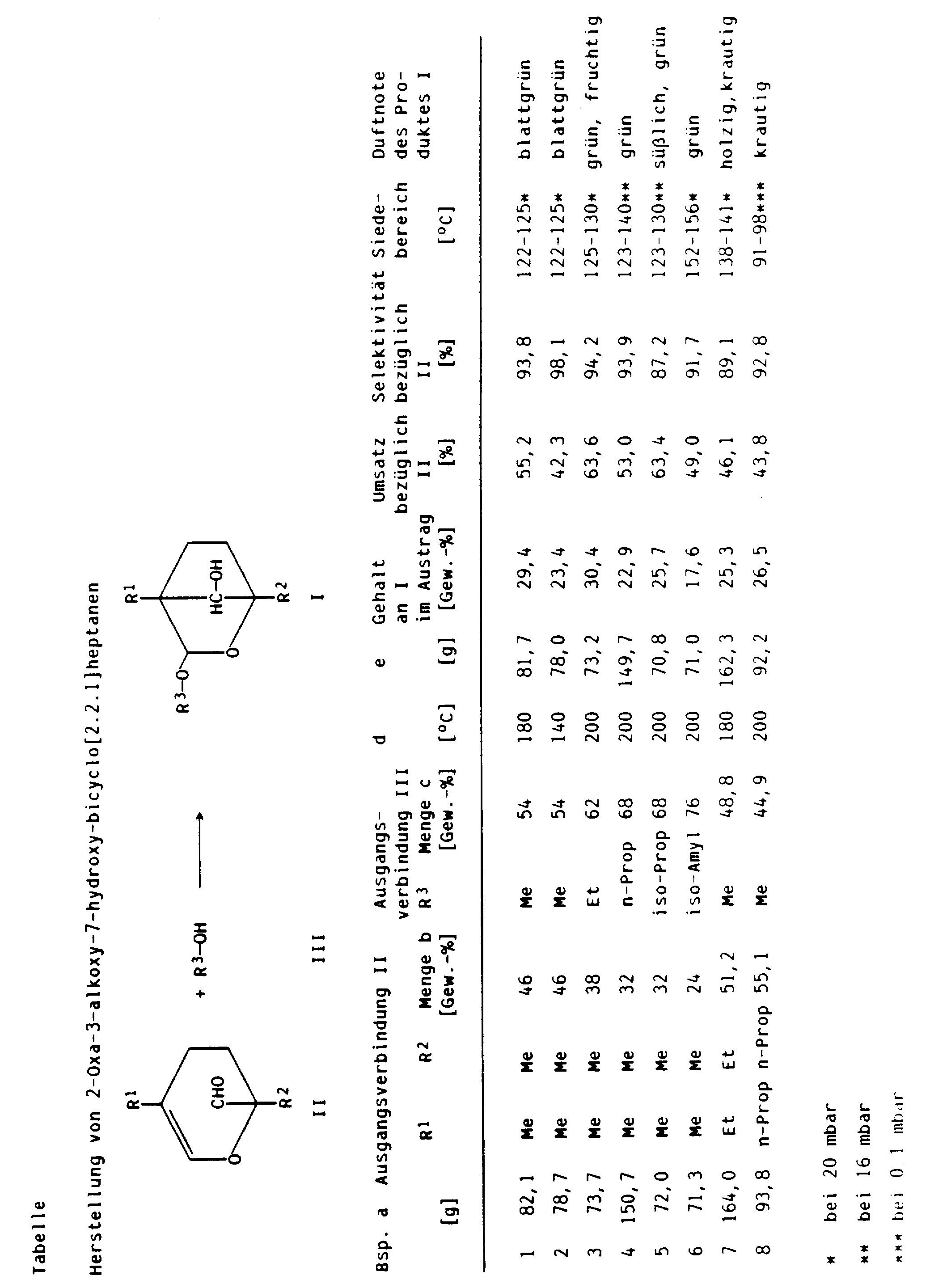

## Patentansprüche

1. 2-oxa-3-alkoxy-7-hydroxy-bicyclo[2.2.1]heptane der allgemeinen Formel I 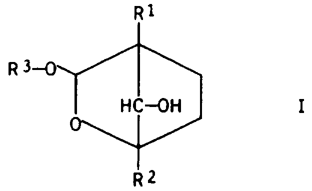 in der die Reste R¹, R und R³ folgende Bedeutung haben:
- C₁-C₁₀-Alkylgruppen, die bis zu drei der folgenden Substituenten R⁴ tragen können:
C₃-C₁₂-Cycloalkyl; Phenyl; Naphthyl; 5- oder 6-gliedrige aromatische oder nichtaromatische Heterocyclen; C₂-C₆-Alkenyl; C₂-C₆-Alkinyl; C₁-C₁₂-Alkoxycarbonyl; C₁-C₁₂-Acyloxy; Amino; Mono-C₁-C₄-alkylamino; Di-C₁-C₄-alkylamino; C₁-C₁₂-Acyl; Di-(C₁-C₄-alkyl)-phosphonyl; Hydroxyl; Cyano; C₁-C₄-Alkoxy; C₁-C₄-Alkylthio; 5- oder 6-gliedrige Cycloalkylgruppen, deren Ringkohlenstoffatome durch nichtbenachbarte Sauerstoff- oder Schwefelatome ersetzt sein können;
- C₃-C₈-Cycloalkylgruppen,
- ein- oder zweikernige Arylreste, wobei diese bis zu drei C₁-C₁₂-Alkylgruppen, C₁-C₁₂-Alkoxygruppen, C₂-C₈-Alkenylgruppen, Chlor- oder Aminoreste als Substituenten tragen können,
- 5- oder 6-gliedrige aromatische oder nichtaromatische Heterocyclen,
und in der die Reste R¹ oder R auch für Wasserstoff stehen können.

2. Verbindungen I nach Anspruch 1, bei denen die Reste R¹, R R2 und R³ folgende Bedeutung haben:
- C₁-C₈-Alkylgruppen, die bis zu drei der folgenden Substituenten R⁴ tragen können:
C₃-C₈-Cycloalkyl; Phenyl; Pyridyl; C₂-C₄-Alkenyl oder C₁-C₈-Alkoxycarbonyl,
und bei denen die Reste R¹ und R die gleiche Bedeutung haben.

3. Verfahren zur Herstellung der Verbindungen I gemäß Anspruch 1 oder 2, dadurch gekennzeichnet, daß man ein 2-Formyl-3,4-dihydropyran der allgemeinen Formel II 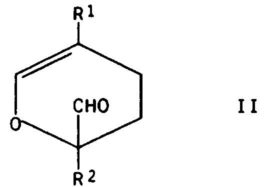 mit einem Alkohol der allgemeinen Formel III
R³-OH III
in Gegenwart eines aciden Heterogenkatalysators bei 20 bis 350°C in der Flüssigphase umsetzt.

4. Verfahren nach Anspruch 3, dadurch gekennzeichnet, daß man als acide Heterogenkatalysatoren saure Oxide oder Phosphate verwendet.

5. Verfahren nach Anspruch 3, dadurch gekennzeichnet, daß man als aciden Heterogenkatalysator Siliciumdioxid einsetzt.

6. Verfahren nach Anspruch 3, dadurch gekennzeichnet, daß man die Umsetzung bei 80 bis 300°C durchführt.

7. Verwendung der Verbindungen I gemäß Anspruch 1 oder 2 als Duftstoffe oder Zwischenprodukte zur Synthese von Wirkstoffen.

8. Kosmetische Zubereitungen sowie Wasch-, Reinigungs- und Pflegemittel, enthaltend die Verbindungen I gemäß Anspruch 1 oder 2 als Duftstoffe.

## Claims

1. A 2-oxa-3-alkoxy-7-hydroxybicyclo[2.2.1]heptane of the formula I 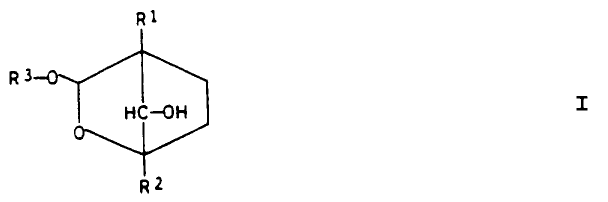
where R¹, R and R³ are each C₁-C₁₀-alkyl which can carry up to three of the following substituents R⁴:
C₃-C₁₂-cycloalkyl; phenyl; naphthyl; 5- or 6-membered aromatic or non-aromatic heterocycles; C₂-C₆-alkenyl; C₂-C₆-alkynyl; C₁-C₁₂-alkoxycarbonyl; C₁-C₁₂-acyloxy; amino; mono-C₁-C₄-alkylamino; di-C₁-C₄-alkylamino; C₁-C₁₂-acyl; di-(C₁-C₄-alkyl)-phosphonyl; hydroxyl; cyano; C₁-C₄-alkoxy; C₁-C₄-alkylthio; 5- or 6-membered cycloalkyl whose ring carbons can be replaced by non-adjacent oxygen or sulfur; or are each C₃-C₈-cycloalkyl,
mononuclear or dinuclear aryl which can be substituted up to three times by C₁-C₁₂-alkyl, C₁-F₁₂-alkoxy, C₂-C₈-alkenyl, chlorine or amino,
5- or 6-membered aromatic or non-aromatic heterocycles, and where R¹ or R can also be hydrogen.

2. A compound I as claimed in claim 1, where R¹, R and R³ are each C₁-C₈-alkyl which can carry up to three of the following substituents R⁴:
C₃-C₈-cycloalkyl; phenyl; pyridyl; C₂-C₄-alkenyl or C₁-C₈-alkoxycarbonyl,
and where R¹ and R have the same meaning.

3. A process for preparing a compound I as claimed in claim 1 or 2, which comprises reacting a 2-formyl-3,4-dihydropyran of the formula II 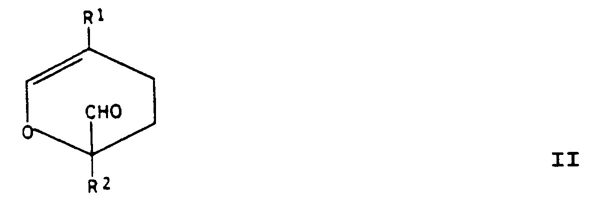 with an alcohol of the formula III
R³-OH III
in the presence of an acidic heterogeneous catalyst at 20 to 350°C in the liquid phase.

4. A process as claimed in claim 3, wherein acidic oxides or phosphates are used as acidic heterogeneous catalysts.

5. A process as claimed in claim 3, wherein silicon dioxide is employed as acidic heterogeneous catalyst.

6. A process as claimed in claim 3, wherein the reaction is carried out at 80 to 300°C.

7. The use of the compounds I as claimed in claim 1 or 2 as fragrances or intermediates for synthesizing active compounds.

8. A cosmetic preparation or detergent, cleaner or material-care agent containing a compound I as claimed in claim 1 or 2 as fragrance.

## Revendications

1. 2-oxa-3-alcoxy-7-hydroxy-bicyclo[2.2.1]heptane de formule générale I 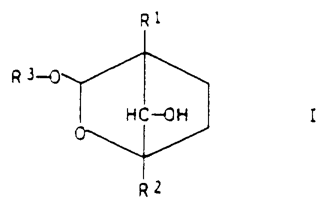 dans laquelle les restes R¹, R et R³ ont les significations suivantes :
- groupes alkyle en C1-C10 qui peuvent porter jusqu'à trois des substituants R⁴ suivants :
cycloalkyle en C3-C12 ; phényle ; naphtyle ; hétérocycles aromatiques ou non aromatiques à 5 ou 6 chaînons ; alcényle en C2-C6 ; alcynyle en C2-C6 ; alcoxy en C1-C12-carbonyle ; acyloxy en C1-C12 ; amino ; mono-alkyle en C1-C4-amino ; di-alkyle en C1-C4-amino ; acyle en C1-C12 ; di-(alkyle en C1-C4)-phosphonyle ; hydroxyle ; cyano ; alcoxy en C1-C4 ; alkylthio en C1-C4 ; groupes cycloalkyle à 5 ou 6 chaînons, dont des atomes de carbone du cycle peuvent être remplacés par des atomes d'oxygène ou de soufre non voisins ;
- groupes cycloalkyle en C3-C8,
- restes aryle à un ou deux noyaux, ceux-ci pouvant porter comme substituants jusqu'à trois groupes alkyle en C1-C12, groupes alcoxy en C1-C12, groupes alcényle en C2-C8, des restes chloro ou amino,
- hétérocycles aromatiques ou non aromatiques à 5 ou 6 chaînons,
et dans lequel les restes R¹ ou R peuvent aussi représenter l'hydrogène.

2. Composés I selon la revendication 1 dans lesquels les restes R¹, R et R³ ont les significations suivantes :
- groupes alkyle en C1-C8, qui peuvent porter jusqu'à trois des substituants R⁴ suivants :
cycloalkyle en C3-C8 ; phényle ; pyridyle ; alcényle en C2-C4 ou alcoxy en C1-C8-carbonyle,
et dans lequel les restes R¹ et R ont la même signification.

3. Procédé de préparation des composés I selon la revendication 1 ou 2, caractérisé par le fait que l'on fait réagir un 2-formyl-3,4-dihydropyrane de formule générale II 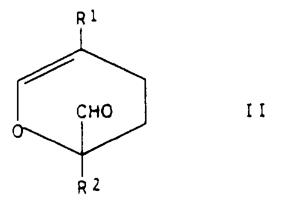 avec un alcool de formule générale III
R³-OH III
en présence d'un catalyseur hétérogène acide à 20 à 350°C en phase liquide.

4. Procédé selon la revendication 3, caractérisé par le fait que l'on utilise, comme catalyseurs hétérogènes acides, des oxydes ou phosphates acides.

5. Procédé selon la revendication 3, caractérisé par le fait que l'on fait usage, comme catalyseur hétérogène acide, du dioxyde de silicium.

6. Procédé selon la revendication 3, caractérisé par le fait que l'on effectue la réaction à 80 à 300°C.

7. Utilisation des composés I selon la revendication 1 ou 2 comme parfum ou produit intermédiaire pour la synthèse de constituants actifs.

8. Préparations cosmétiques ainsi qu'agents de lavage, de nettoyage et d'entretien contenant, comme parfum, les composés I selon les revendications 1 ou 2.
